(19) **Europäisches Patentamt / European Patent Office / Office européen des brevets**

(11) **EP 4 076 732 B1**

(12) **FASCICULE DE BREVET EUROPÉEN**

(45) Date de publication et mention de la délivrance du brevet:
**12.02.2025 Bulletin 2025/07**

(21) Numéro de dépôt: **20848849.4**

(22) Date de dépôt: **18.12.2020**

(51) Classification Internationale des Brevets (IPC):
**B01J 20/18** (2006.01)  **B01J 20/28** (2006.01)
**B01J 20/30** (2006.01)

(52) Classification Coopérative des Brevets (CPC):
**B01J 20/18; B01J 20/28011; B01J 20/2803;
B01J 20/28042; B01J 20/3028; B01J 20/3078;
B01J 20/3085;** Y02P 20/52

(86) Numéro de dépôt international:
**PCT/FR2020/052532**

(87) Numéro de publication internationale:
**WO 2021/123664 (24.06.2021 Gazette 2021/25)**

(54) **ADSORBANT ZÉOLITHIQUE POUR LA SÉPARATION D'ISOMÈRES D'HYDROCARBURES**

ZEOLITHADSORBENS ZUR TRENNUNG VON KOHLENWASSERSTOFFISOMEREN

ZEOLITE ADSORBENT FOR THE SEPARATION OF HYDROCARBON ISOMERS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.12.2019 FR 1915324**

(43) Date de publication de la demande:
**26.10.2022 Bulletin 2022/43**

(73) Titulaire: **ARKEMA FRANCE
92700 Colombes (FR)**

(72) Inventeurs:
- **PEREZ-PELLITERO, Javier
  92852 Rueil-Malmaison Cedex (FR)**
- **BOUVIER, Ludivine
  64170 Lacq (FR)**
- **MANKO, Maria
  92852 Rueil-Malmaison Cedex (FR)**

(56) Documents cités:
**FR-A1- 2 998 808     US-B2- 10 125 064**

EP 4 076 732 B1

**Description**

DOMAINE TECHNIQUE

L'invention concerne le domaine des adsorbants zéolithiques sous forme d'agglomérés comprenant de la zéolithe de type faujasite, pour la séparation de mélanges gazeux ou liquides d'hydrocarbures aromatiques, et concerne plus particulièrement les procédés de séparation des xylènes et notamment des procédés de séparation de *méta*-xylène à productivité améliorée.

La présente invention concerne également un procédé de séparation de mélanges gazeux ou liquides d'isomères à productivité améliorée, et plus particulièrement un procédé de séparation des isomères du xylène à productivité améliorée pour la production de *méta*-xylène très pur à partir d'une charge d'hydrocarbures aromatiques contenant des isomères à 8 atomes de carbone.

TECHNIQUE ANTERIEURE

L'utilisation d'adsorbants zéolithiques constitués de zéolithes Faujasite (FAU) de type Y pour adsorber sélectivement le *méta*-xylène dans un mélange d'hydrocarbures aromatiques, est bien connue de l'art antérieur.

Par exemple, les brevets US4306107, US4326092, US5382747, US5900523 et US7728187 ainsi que FR2998808, FR2889698 et FR2889699 montrent que des adsorbants zéolithiques comprenant des aluminosilicates à base de sodium ou à base de sodium et de lithium, sont efficaces pour la séparation du *méta*-xylène présent dans des coupes aromatiques en C8 (coupes comprenant des hydrocarbures aromatiques à 8 atomes de carbone). Le brevet TW201107032 décrit des adsorbants à base de zéolithe Y échangée à l'argent ou au cuivre ayant des propriétés de transfert et de sélectivité améliorées pour la récupération du *méta*-xylène.

Les adsorbants décrits dans le brevet US5900523 sont utilisés comme agents d'adsorption dans les procédés en phase liquide, de préférence de type contre-courant simulé, similaires à ceux décrits dans le brevet US2985589 et qui s'appliquent, entre autres, aux coupes aromatiques en C8.

L'invention a pour but d'améliorer la productivité des procédés de préparation de *méta*-xylène existants et notamment les procédés en phase liquide, de préférence de type contre-courant simulé de séparation des isomères de xylène à partir de charges aromatiques en C8. Il a été observé de manière surprenante que cette productivité peut être améliorée par le choix judicieux des caractéristiques des agglomérés adsorbants zéolithiques mis en œuvre dans ce type de procédés.

La séparation en lit mobile simulé s'entend ici au sens large, c'est-à-dire qu'on peut avoir affaire soit à un lit mobile à contre-courant simulé, soit à un lit mobile à co-courant simulé, soit à un procédé dit "Varicol". Le procédé Varicol proposé par Ludemann-Hombourger (O. Ludemann-Hombourger, R. Nicoud, 2000) et développé plus tard par l'entreprise Novasep, permet un déplacement non synchronisé des points d'injection et de soutirage (Bailly, *et al.* 2004). Les longueurs des quatre zones peuvent être ajustées pendant le cycle en limitant le nombre de lits nécessaire pour réaliser la séparation.

La caractéristique commune à cette famille de procédés est que l'aggloméré adsorbant zéolithique (ou plus simplement « solide adsorbant ») est mis en œuvre en lit fixe, et que les flux liquides en contact avec le solide adsorbant sont gérés soit au moyen d'un jeu de vannes « tout ou rien », soit au moyen d'une vanne unique complexe connue sous le nom de vanne rotative ou "rotary valve".

Lorsque l'élément actif des solides adsorbants utilisés comme agents d'adsorption dans ces procédés est une zéolithe, cette dernière, obtenue sous forme de cristaux, est de préférence employée à l'échelle industrielle sous forme d'agglomérés. Ces adsorbants zéolithiques, agglomérés sous forme de plaquettes, billes ou extrudés, sont en général constitués de cristaux de zéolithe, qui constitue l'élément actif au sens de l'adsorption et d'un liant destiné à assurer la cohésion des cristaux sous forme d'agglomérés. Ce liant confère également aux agglomérés une résistance mécanique suffisante pour résister aux contraintes mécaniques auxquels ils sont soumis au cours de leur mise en œuvre au sein des unités. Ces contraintes mécaniques sont à l'origine de la formation de « fines », qui induisent une détérioration des performances au cours de la durée d'opération du procédé.

Le procédé de séparation des xylènes en lit mobile simulé (LMS) a connu de très nombreuses améliorations technologiques, notamment au niveau des plateaux distributeurs des fluides, mais relativement peu d'évolution concernant les caractéristiques intrinsèques du solide adsorbant.

Le document US4306107 décrit l'utilisation, en tant qu'adsorbant sélectif du *méta*-xylène, d'une zéolithe Y, dans laquelle les sites cationiques échangeables sont occupés par des atomes de sodium. Pour obtenir une sélectivité satisfaisante en faveur du *méta*-xylène, il est recommandé d'utiliser une zéolithe partiellement hydratée, présentant une perte au feu à 950°C de 2% à 7% en poids du poids initial de l'adsorbant. Il est préconisé dans ce document une séparation par un procédé en lit mobile simulé à une température comprise entre 20°C et 250°C et à une pression comprise entre la pression atmosphérique et 3,5 MPa (35 bars), valeur choisie de manière à maintenir la charge sous forme liquide.

L'occupation des sites échangeables de la zéolithe par des ions sodium et l'activation afin d'obtenir la perte au feu souhaitée permettent d'obtenir des agglomérés qui présentent, du point de vue de l'adsorption du *méta*-xylène contenu dans les coupes aromatiques en C8, des propriétés améliorées de capacité et de sélectivité.

Les adsorbants pour la séparation des xylènes présentant des propriétés de transfert améliorées pour la séparation des xylènes sont par exemple décrits dans la demande internationale WO2008009845 qui décrit des adsorbants à base de zéolithe X, à petits cristaux, de taille inférieure à 1,7 μm, de rapport atomique Si/Al, tel que 1,15 < Si/Al ≤ 1,5, échangés au baryum, et éventuellement au potassium, dans la demande internationale WO2014090771, qui décrit des adsorbants zéolithiques agglomérés, à propriétés optimisées, notamment pour la séparation du *para*-xylène des coupes aromatiques en C8. Ces adsorbants présentent des propriétés maximales de sélectivité vis-à-vis du *para*-xylène et de transfert de matière, tout en présentant une résistance mécanique maximale associée à une capacité d'adsorption optimisée.

La demande internationale WO2018002174 propose des adsorbants zéolithiques sous forme d'agglomérés aux propriétés optimisées pour la séparation de mélanges gazeux ou liquides d'isomères et plus particulièrement pour la séparation des xylènes, en phase gaz ou en phase liquide, notamment du para-xylène des coupes aromatiques en C8. Les adsorbants zéolithiques de l'invention présentent notamment des propriétés maximales de sélectivité vis-à-vis du *para*-xylène et de transfert de matière, tout en présentant une résistance améliorée et une capacité d'adsorption par volume d'adsorbant élevées et sont particulièrement adaptés pour une utilisation dans un procédé de séparation du *para*-xylène en phase liquide, de préférence de type contre-courant simulé.

En particulier, cette demande enseigne qu'il n'est pas souhaitable d'augmenter fortement la macroporosité et/ou mésoporosité, donc la porosité de grain car cette porosité ne participe pas à la capacité d'adsorption. L'optimisation des propriétés de diffusion et des capacités d'adsorption optimales, ont été obtenues en sélectionnant de manière spécifique à la fois la porosité et le facteur de tortuosité.

La demande de brevet US2009/0326308 décrit un procédé de séparation au moyen d'un adsorbant à faible teneur en liant et à base de cristaux de faujasite de type X de taille nanométrique, typiquement de taille moyenne inférieure à 500 nm.

Dans le procédé de séparation des xylènes par adsorption en lit mobile simulé, l'adsorbant zéolithique est mis en contact avec le flux liquide d'alimentation (charge) comprenant le plus souvent des mélanges d'hydrocarbures en C8, et généralement et le plus souvent des mélanges d'isomères du xylène et plus particulièrement des mélanges de *ortho*-xylène, *méta*-xylène, *para*-xylène et éthylbenzène.

En utilisant un adsorbant zéolithique à base de zéolithe de structure faujasite de type Y échangée au sodium ou échangée au sodium et lithium, le *méta*-xylène est alors adsorbé dans les micropores de la zéolithe préférentiellement par rapport à l'ensemble des autres composés hydrocarbures présents dans le flux d'alimentation. La phase adsorbée dans les micropores de la zéolithe se trouve alors enrichie en *méta*-xylène par rapport au mélange initial constituant le flux d'alimentation. La phase liquide se trouve à l'inverse enrichie en composés tels que, *ortho-xylène,* para-xylène et éthylbenzène en proportion relative plus importante que celle caractérisant le mélange initial constituant le flux d'alimentation.

La phase liquide est soustraite du contact avec l'adsorbant formant ainsi un flux de raffinat. La phase adsorbée, enrichie en *méta*-xylène, est désorbée sous l'action d'un flux de désorbant, et soustraite du contact avec l'adsorbant formant alors un flux d'extrait.

Dans le procédé de séparation des xylènes par adsorption en lit mobile simulé, le solide adsorbant zéolithique est mis en œuvre dans une ou deux colonnes multi-étagées pour être mis en contact avec le flux de liquide. On appelle colonne multi-étagée une colonne constituée d'une multiplicité de plateaux disposés selon un axe sensiblement vertical, chaque plateau supportant un lit de solide granulaire, et les différents lits successifs étant traversés en série par le ou les fluides mis en œuvre dans la colonne. Entre deux lits successifs est situé un dispositif de distribution des fluides permettant d'alimenter chaque lit de solide granulaire.

De manière générale le fonctionnement d'une colonne en lit mobile simulé peut être décrit de la façon suivante:

Une colonne comporte au moins quatre zones, et éventuellement cinq ou six, chacune de ces zones étant constituée par un certain nombre de lits successifs, et chaque zone étant définie par sa position comprise entre un point d'alimentation et un point de soutirage. Typiquement, une unité CCS pour la production de *méta*-xylène est alimentée par au moins une charge F à fractionner (charge d'hydrocarbures aromatiques constituée des isomères à 8 atomes de carbones) et un désorbant D, parfois appelé éluant, et l'on soutire de ladite unité au moins un raffinat R contenant les produits de la charge les moins sélectivement adsorbés et du désorbant et un extrait E contenant le produit de la charge le plus adsorbé et du désorbant.

D'autres points d'injection et de soutirage peuvent être ajoutés de manière à rincer les circuits de distribution, comme décrit par exemple dans le brevet US7208651. L'ajout de ces flux supplémentaires de rinçage ne changeant en rien le principe de fonctionnement du CCS, dans un souci de concision, nous n'ajouterons pas ces points d'injection et de soutirage supplémentaires dans la description du procédé selon l'invention.

Les points d'alimentation et de soutirage sont modifiés au cours du temps, décalés dans le même sens d'une valeur correspondant à un lit. Les décalages des différents points d'injection ou de soutirage peuvent être soit simultanés, soit non-simultanés comme l'enseigne le brevet US6136198. Le procédé selon ce second mode de fonctionnement est appelé Varicol.

Classiquement, on définit 4 zones chromatographiques différentes dans une colonne fonctionnant en contre-courant simulé (CCS).

Zone 1 : zone de désorption du produit de la charge le plus adsorbé, comprise entre l'injection du désorbant D et le prélèvement de l'extrait E.

Zone 2 : zone de désorption des produits de la charge les moins sélectivement adsorbés, comprise entre le prélèvement de l'extrait E et l'injection de la charge à fractionner F.

Zone 3 : zone d'adsorption du produit de la charge le plus adsorbé, comprise entre l'injection de la charge et le soutirage du raffinat R.

Zone 4 : zone située entre le soutirage de raffinat R et l'injection du désorbant D.

Pour augmenter la productivité du procédé de séparation, l'art antérieur enseigne qu'une manière consiste à améliorer le transfert global dans l'aggloméré adsorbant zéolithique et notamment en diminuant la taille des cristaux et/ou la taille moyenne desdits agglomérés.

Les documents de l'art antérieur décrivant des procédés de séparation pharmaceutique (Gomes et al., (2006), Adsorption, vol. 12, p. 375 sqq.) rapportent des procédés de séparation chromatographique en phase liquide utilisant des agglomérés de quelques dizaines de micromètres à 100 $\mu$m.

Dans ces procédés utilisant des adsorbants de très petite taille, la perte de charge $\Delta P$ est alors élevée. Dans les procédés de séparation des xylènes, de tels niveaux de perte de charge $\Delta P$ ne sont pas courants. On remarque néanmoins de façon étonnante que la perte de charge $\Delta P$ n'est pas un critère dimensionnant. Il aura un impact notamment sur l'épaisseur des parois de l'adsorbeur et sur la puissance des installations.

Une autre caractéristique de l'aggloméré adsorbant zéolithique est le taux d'hydratation dudit aggloméré. Le maintien de l'hydratation de la zéolithe à la valeur souhaitée, par exemple une perte au feu inférieure ou égale à 7%, pour la zéolithe Y, lors de sa mise en œuvre dans le procédé de séparation des xylènes par adsorption en lit mobile simulé, est assuré en ajoutant de l'eau dans les flux d'alimentation de la charge et/ou de désorbant. L'addition d'eau nécessaire pour de tels niveaux de perte au feu est telle que la teneur en eau en poids dans les effluents hydrocarbonés (le flux d'extrait ou de raffinat) est le plus souvent comprise entre 0 ppm et 150 ppm, et plus généralement entre 0 et 80 ppm lorsque l'adsorbant est à base de zéolithe FAU-Y.

Néanmoins il est toujours nécessaire d'augmenter la productivité du procédé de manière générale.

De tels matériaux adsorbants zéolithiques à base de zéolithe de type FAU-Y, et en particulier contenant des cations choisis parmi lithium, potassium, sodium, baryum, calcium, et les mélanges de deux ou plusieurs d'entre eux, pourraient apporter des améliorations significatives dans les procédés de séparation des xylènes présents sous forme de mélanges d'isomères dans les coupes d'hydrocarbures aromatiques en C8, et en particulier pourraient grandement améliorer l'adsorption sélective de *méta*-xylène dans les mélanges d'hydrocarbures aromatiques en C8.

La présente invention a ainsi pour premier objet de proposer un adsorbant zéolitique sous forme d'agglomérés aux propriétés optimisées pour la séparation de mélanges gazeux ou liquides d'isomères et plus particulièrement pour la séparation des xylènes, en phase gaz ou en phase liquide, notamment pour la séparation du *méta*-xylène à partir de coupes aromatiques en C8. Les agglomérés adsorbants zéolitiques de l'invention présentent notamment des propriétés maximales de sélectivité vis-à-vis du *méta*-xylène et de transfert de matière, tout en présentant une résistance mécanique et une capacité d'adsorption élevées et sont particulièrement adaptés pour une utilisation dans un procédé de séparation du *méta*-xylène en phase liquide, de préférence de type contre-courant simulé.

La présente invention propose à cet effet un adsorbant aggloméré, préférentiellement à base de zéolithe faujasite de rapport atomique Si/Al strictement supérieur à 1,50, avantageusement compris entre 1,50 et 6,50 bornes exclues, et dont la porosité de grain est avantageusement comprise entre 25% et 45%, permettant la production de *méta*-xylène de haute pureté avec une productivité améliorée, tout en évitant la dégradation des performances au cours du temps. Plus précisément, l'invention concerne un adsorbant aggloméré zéolithique, comprenant au moins une zéolithe faujasite de rapport atomique Si/Al strictement supérieur à 1,50, avantageusement compris entre 1,50 et 6,50 bornes exclues, de préférence entre 2,00 et 6,00 bornes incluses, de préférence encore entre 2,00 et 4,00, bornes incluses et de manière encore plus préférée entre 2,20 et 2,80 bornes incluses, et dans lequel d'une part la porosité de grain est comprise entre 25% et 45 %, de préférence entre 30% et 45 %, de préférence encore entre 32% et 45% et de manière très préférée entre 35% et 45%, de manière particulièrement avantageuse entre 36% et 45%, bornes incluses, et d'autre part en ce que la déviation standard $\sigma$ de la distribution de taille de cristaux dans ledit aggloméré est inférieure à 0,30 $\mu$m, de préférence comprise entre 0,05 $\mu$m et 0,30 $\mu$m, de préférence encore entre 0,05 $\mu$m et 0,28 $\mu$m, et de manière tout à fait préférée entre 0,1 $\mu$m et 0,28 $\mu$m, de préférence entre toutes entre 0,1 $\mu$m et 0,25 $\mu$m, bornes incluses, ledit adsorbant comprenant des cristaux de zéolithe de diamètre moyen en nombre compris entre 100 nm et 1200 nm, bornes incluses, et la fraction massique de zéolithe FAU dans ledit adsorbant est supérieure ou égale à 80% par rapport au poids total d'adsorbant.

RESUME DE L'INVENTION

Ainsi, et selon un premier aspect, l'invention concerne un adsorbant zéolithique aggloméré, comprenant au moins une zéolithe faujasite de rapport atomique Si/Al strictement supérieur à 1,50, avantageusement compris entre 1,50 et 6,50 bornes exclues, de préférence entre 2,00 et 6,00 bornes incluses, de préférence encore entre 2,00 et 4,00, bornes incluses et de manière encore plus préférée entre 2,20 et 2,80 bornes incluses, caractérisé en ce que la porosité de grain dudit adsorbant est comprise entre 25% et 45 %, de préférence entre 30% et 45 %, de préférence encore entre 32% et 45% et de manière très préférée entre 35% et 45%, de manière particulièrement avantageuse entre 36% et 45%, bornes incluses, et d'autre part en ce que la déviation standard σ de la distribution de taille de cristaux dans ledit aggloméré est inférieure à 0,30 μm, de préférence comprise entre 0,05 μm et 0,30 μm, de préférence encore entre 0,05 μm et 0,28 μm, et de manière tout à fait préférée entre 0,1 μm et 0,28 μm, de préférence entre toutes entre 0,1 μm et 0,25 μm, bornes incluses, ledit adsorbant comprenant des cristaux de zéolithe de diamètre moyen en nombre compris entre 100 nm et 1200 nm, bornes incluses, et la fraction massique de zéolithe FAU dans ledit adsorbant est supérieure ou égale à 80% par rapport au poids total d'adsorbant.

Selon un mode de réalisation, l'adsorbant zéolithique aggloméré selon l'invention comprend des cristaux de zéolithe de diamètre moyen en nombre de préférence compris entre 400 nm et 1200 nm, plus préférentiellement entre 500 nm et 1200 nm, de manière tout à fait préférée entre 550 nm et 1200 nm, très avantageusement entre 600 nm et 1200 nm, bornes incluses.

Selon un autre mode de réalisation, l'adsorbant zéolithique aggloméré selon l'invention se présente sous la forme de billes de diamètre moyen compris entre 100 μm et 1000 μm, de préférence entre 100 μm et 600 μm, de préférence encore 200 μm et 550 μm, bornes incluses.

Dans un autre mode de réalisation préféré de l'invention, aucune structure zéolithique autre que la structure faujasite, de préférence aucune structure zéolithique autre que la structure faujasite Y, n'est détectée par diffraction des rayons X, dans l'adsorbant zéolithique aggloméré selon l'invention.

Par « aucune », on entend moins de 5% en masse, de préférence moins de 2% en masse, de zéolithe de structure autre que structure FAU, par rapport à la masse totale de l'adsorbant, les fractions massiques étant déterminées par DRX.

En outre, la fraction massique de zéolithe FAU, et de préférence la fraction massique de zéolithe FAU-Y, est supérieure ou égale à 80% par rapport au poids total d'adsorbant zéolithique aggloméré selon l'invention, le complément à 100% étant de préférence constitué de phase non zéolithique, c'est-à-dire une phase non cristalline qui est essentiellement inerte vis-à-vis de l'adsorption.

L'adsorbant zéolithique aggloméré selon l'invention comprend un ou plusieurs cation(s), de préférence alcalin(s) ou alcalino-terreux, de préférence encore choisi(s) parmi lithium, sodium, baryum, calcium et potassium, seuls ou en mélanges de deux ou plusieurs d'entre eux, et de manière tout à fait préférée choisi(s) parmi lithium, sodium et calcium, seuls ou en mélanges de deux ou plusieurs d'entre eux.

Selon un mode de réalisation préféré, la teneur en sodium, exprimée en oxyde de sodium ($Na_2O$) dans l'adsorbant zéolithique aggloméré selon l'invention est supérieure à 5%, de préférence encore supérieure à 8%, de manière très préférée supérieure à 10%, en poids par rapport à la masse totale de l'adsorbant, et avantageusement, ladite teneur en oxyde de sodium est comprise entre 10% et 17%, et typiquement entre 11% et 13%, bornes incluses, en poids par rapport au poids total de l'adsorbant.

Selon un autre mode de réalisation préféré, la teneur en potassium, exprimée en oxyde de potassium ($K_2O$) dans l'adsorbant zéolithique aggloméré selon l'invention est inférieure à 12%, de préférence inférieure à 5%, de préférence comprise entre 0 et 3%, de manière encore plus préférée comprise entre 0 et 2%, bornes incluses, en poids par rapport à la masse totale de l'adsorbant.

Selon un autre mode de réalisation préféré, la teneur en calcium, exprimée en oxyde de calcium (CaO) dans l'adsorbant zéolithique aggloméré selon l'invention est inférieure à 6%, de préférence inférieure à 5%, de préférence comprise entre 0 et 4%, de manière encore plus préférée comprise entre 0 et 3%, bornes incluses, en poids par rapport à la masse totale de l'adsorbant.

Selon un mode de réalisation de l'invention, l'adsorbant zéolithique peut présenter une teneur en baryum, exprimée en oxyde de baryum inférieure à 6%, de préférence comprise entre 0 et 4%, bornes incluses, exprimée en poids d'oxyde de baryumBaO par rapport à la masse totale de l'adsorbant.

Selon un mode de réalisation de l'invention, l'adsorbant zéolithique peut présenter une teneur en lithium inférieure à 8%, de préférence comprise entre 0 et 4%, bornes incluses, exprimée en poids d'oxyde de lithium $Li_2O$ par rapport à la masse totale de l'adsorbant.

Selon un autre mode de réalisation de l'invention la teneur totale en cations autres que le lithium, le sodium, le potassium, le calcium et le baryum est généralement inférieure à 2% et le plus souvent comprise entre 0 et 1 %, bornes incluses, cette teneur totale étant exprimée en masse d'oxydes desdits cations, par rapport à la masse totale de l'adsorbant zéolithique.

Dans un mode de réalisation de la présente invention, la perte au feu de l'adsorbant zéolithique aggloméré selon l'invention, mesurée à 900°C selon la norme NF EN 196-2, est inférieure ou égale à 7%, de préférence comprise entre 0 et 6%, de manière préférée entre 0 et 4%, de manière encore préférée entre 0 et 3% et avantageusement entre 0 et 2,5%, bornes incluses.

Selon un autre aspect, la présente invention concerne l'utilisation de l'adsorbant zéolithique aggloméré tel qu'il vient d'être décrit dans les procédés de :

- séparation de coupes d'isomères aromatiques en C8 et notamment des xylènes, et plus particulièrement de méta-xylène,
- séparation d'isomères de toluène substitué tels que nitrotoluène, diéthyltoluène, toluènediamine, et autres,
- séparation des crésols,
- séparation des alcools polyhydriques.

Enfin, et selon encore un autre aspect, la présente invention concerne le procédé de séparation de *méta*-xylène à partir de coupes d'isomères aromatiques à 8 atomes de carbone, utilisant comme agent d'adsorption du *méta*-xylène, un adsorbant zéolithique aggloméré tel qu'il a été défini précédemment et plus précisément dans la suite du présent exposé, en phase liquide ou en phase gazeuse.

Le procédé de séparation de *méta*-xylène à partir de coupes d'isomères d'hydrocarbures aromatiques contenant 8 atomes de carbone, de la présente invention, est de préférence conduit en phase liquide, par adsorption du *méta*-xylène en présence d'un désorbant, ledit désorbant pouvant être tout désorbant connu de l'homme du métier et notamment tout désorbant dont le point d'ébullition est inférieur à celui de la charge, tel que le toluène ou l'indane mais aussi un désorbant dont le point d'ébullition est supérieur à celui de la charge, tel que la tétraline.

Selon un mode de réalisation préféré, le procédé de séparation de *méta*-xylène selon la présente invention, est un procédé de type lit mobile simulé, de préférence encore de type à contre-courant simulé.

DESCRIPTION DES MODES DE REALISATION

L'adsorbant zéolithique de l'invention comprend de préférence à la fois des macropores, des mésopores et des micropores. Par « macropores », on entend des pores dont l'ouverture est supérieure à 50 nm, de préférence comprise entre 50 nm et 400 nm. Par « mésopores », on entend des pores dont l'ouverture est comprise entre 2 nm et 50 nm, bornes non incluses. Par « micropores », on entend des pores dont l'ouverture est inférieure à 2 nm.

Comme indiqué précédemment, l'adsorbant selon la présente invention se présente sous forme d'un adsorbant dont la porosité de grain est comprise entre 25% et 45 %, de préférence entre 30% et 45 %, de préférence encore entre 32% et 45%, de manière très préférée entre 35% et 45%, et de manière particulièrement avantageuse entre 36% et 45% bornes incluses. En outre, la déviation standard $\sigma$ de la distribution de taille de cristaux dans ledit adsorbant est inférieure à 0,30 $\mu$m, de préférence comprise entre 0,05 $\mu$m et 0,30 $\mu$m, de préférence encore entre 0,05 $\mu$m et 0,28 $\mu$m, et de manière tout à fait préférée entre 0,1 $\mu$m et 0,28 $\mu$m, de préférence entre toutes entre 0,1 $\mu$m et 0,25 $\mu$m, bornes incluses.

En effet, les inventeurs ont découvert de manière surprenante que lorsque la déviation standard $\sigma$ de la distribution de taille de cristaux dans l'adsorbant zéolithique est supérieure à 0,3 $\mu$m, on observe une chute drastique de la productivité dans un procédé de séparation de méta-xylène, notamment dans un procédé de séparation en phase liquide, à contre-courant en lit mobile simulé.

Il a également été observé de manière surprenante que cette valeur de productivité atteint un maximum pour des valeurs de déviation standard $\sigma$ de la distribution de taille de cristaux dans l'aggloméré adsorbant zéolithique inférieure à 0,3 $\mu$m. Cette valeur de déviation standard $\sigma$ paraît correspondre à une porosité de grain optimisée. En effet, il a été également observé de façon tout à fait surprenante que la porosité de grain est inversement proportionnelle à la déviation standard $\sigma$ de la distribution de taille de cristaux dans l'aggloméré adsorbant zéolithique. Ainsi, plus la déviation standard $\sigma$ diminue, plus la porosité de grain augmente. Or une porosité de grain trop importante conduit à des effets tout à fait non désirés, tels que par exemple perte de capacité d'adsorption, perte en résistance mécanique, et autres.

Par conséquent, l'homme du métier souhaitant une productivité maximale trouve grâce à la présente invention un compromis entre la porosité du grain et la déviation standard $\sigma$ de la distribution de taille de cristaux dans l'aggloméré adsorbant zéolithique. Grâce à l'adsorbant aggloméré selon l'invention, il est ainsi possible d'obtenir une productivité maximale dans les procédés de séparation des xylènes, notamment pour la séparation de *méta*-xylène.

Avantageusement, l'adsorbant zéolithique aggloméré se présente sous la forme de billes de diamètre moyen compris entre 100 $\mu$m et 1000 $\mu$m, de préférence entre 100 $\mu$m et 600 $\mu$m, de préférence encore 200 $\mu$m et 550 $\mu$m, bornes incluses.

De préférence, l'adsorbant zéolithique à base de faujasite selon l'invention comprend du sodium ou du lithium ou du calcium ou un mélange de deux ou des trois d'entre eux.

De préférence l'adsorbant zéolithique selon la présente invention est un adsorbant à base de zéolithe(s) FAU, généralement référencée(s) sous le nom de zéolithe de type Y. Par « zéolithe Y », on entend une zéolithe dont le rapport atomique Si/Al est compris entre 1,5 et 6,0 bornes exclues, comme indiqué précédemment.

Les agglomérés adsorbants zéolithiques selon la présente invention peuvent contenir une phase non zéolithique (PNZ), c'est-à-dire une phase non cristalline qui est essentiellement inerte vis-à-vis de l'adsorption. Le taux de cristallinité (fraction massique de zéolithe) de l'adsorbant selon l'invention peut être mesuré par analyse par diffraction de rayons X, connue de l'homme du métier sous l'acronyme DRX.

L'aggloméré adsorbant zéolithique de l'invention est de préférence sous la forme d'un aggloméré, c'est-à-dire qu'il est constitué d'éléments cristallins (ou cristaux) d'au moins une zéolithe FAU telle que définie précédemment, lesdits éléments cristallins (ou plus simplement « cristaux ») présentant un diamètre moyen en nombre compris entre 100 nm et 1200 nm, de préférence compris entre 400 nm et 1200 nm, plus préférentiellement entre 500 nm et 1200 nm, de manière tout à fait préférée entre 550 nm et 1200 nm, très avantageusement entre 600 nm et 1200 nm, bornes incluses.

MODES DE PREPARATION

Les agglomérés adsorbants zéolithiques selon la présente invention peuvent être préparés en adaptant les modes opératoires déjà connus de l'homme du métier, et comme décrit par exemple dans le document US2009326308, US10125064, TW201107032 et US2007224113, et en sélectionnant et ajustant les paramètres de synthèse permettant d'obtenir les agglomérés présentant les valeurs souhaitées de porosité de grain et de déviation standard σ.

Un procédé de synthèse de l'aggloméré adsorbant zéolithique selon la présente invention peut par exemple comprendre au moins les étapes de :

a) agglomération de cristaux d'au moins une zéolithe de type FAU-Y, avec un liant comprenant au moins 80% d'argile ou d'un mélange d'argiles zéolithisable(s), et avec éventuellement jusqu'à 5% d'additifs ainsi qu'avec la quantité d'eau qui permet la mise en forme du matériau aggloméré ; séchage des agglomérats à une température comprise entre 50°C et 150°C ; calcination des agglomérats séchés sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température supérieure à 150°C, typiquement comprise entre 180°C et 800°C, préférentiellement entre 200°C et 650°C,

b) zéolithisation éventuelle de tout ou partie du liant par mise en contact des agglomérats obtenus à l'étape a) avec une solution basique alcaline,

c) échange(s) cationique(s) éventuel(s) des agglomérats de l'étape a) et/ou de l'étape b) par mise en contact avec une ou plusieurs solution(s) de cations, de manière simultanée ou séquencée,

d) lavage et séchage des agglomérats obtenus à l'étape c), à une température comprise entre 50°C et 150°C, et

e) activation par chauffage à une température en général comprise entre 100°C et 400°C, de préférence entre 200°C et 300°C puis récupération de l'adsorbant aggloméré zéolithique.

Les cristaux de zéolithes utilisables dans l'étape a) de synthèse ci-dessus peuvent avantageusement être synthétisés selon les modes opératoires connus et disponibles dans la littérature scientifique ou la littérature brevets, ainsi que sur l'internet. On peut en particulier préparer les cristaux de zéolithes comme décrit par exemple dans le document US10071914 et US2007224113.

Les paramètres qui peuvent permettre de contrôler la déviation standard σ dans l'aggloméré adsorbant zéolithique de la présente invention sont par exemple la nature même des cristaux utilisés à l'étape a), notamment la taille et la déviation standard desdits cristaux, mais aussi les conditions de zéolithisation du liant d'agglomération, comme par exemple la température, la durée, le pH de la solution alcaline de zéolithisation, ou encore la durée, le mode d'agitation, le taux de cisaillement, pression, et autres.

Plus spécifiquement par « nature des cristaux utilisés à l'étape a) », on entend notamment la déviation standard desdits cristaux qui peut par exemple être contrôlée en ajustant les paramètres de synthèse, et en particulier la température de synthèse, la vitesse d'agitation, le taux de cisaillement, comme indiqué par exemple dans les documents WO2009081022 ou encore US2009326308.

Les paramètres de synthèse pouvant permettre de contrôler la porosité de l'aggloméré adsorbant zéolithique de l'invention sont également connus de l'homme du métier. De manière générale, ces paramètres comprennent, de manière non limitative le taux de liant, le type d'agglomération (par extrusion, atomisation, granulation, etc.), le taux d'humidité, la nature du liant, les conditions de zéolithisation (température, durée, pH de la solution alcaline de zéolithisation, durée, mode d'agitation, taux de cisaillement, pression et autre)

Selon un mode de réalisation préféré, la synthèse de l'aggloméré adsorbant zéolithique de la présente invention ne comprend pas l'ajout d'agent porogène, la présence d'un agent porogène pouvant entraîner notamment une dégradation de la cristallinité.

Il est également possible de préparer lesdits éléments cristallins par synthèse par ensemencement et/ou par ajustement des conditions opératoires de synthèse tels que le rapport $SiO_2/Al_2O_3$, la teneur en sodium et l'alcalinité du mélange de synthèse.

La synthèse de zéolithe de type FAU se fait généralement en milieu sodique (cation $Na^+$). Les éléments cristallins de zéolithe FAU ainsi obtenus comprennent majoritairement, voire exclusivement des cations sodium. On ne sortirait toutefois pas du cadre de l'invention en utilisant des éléments cristallins ayant subi un ou plusieurs échanges cationiques, entre la synthèse sous forme sodique.

La taille des cristaux de zéolithe FAU utilisés à l'étape a) et des éléments cristallins de zéolithe FAU dans les agglomérés selon l'invention est mesurée par observation au microscope électronique à balayage (MEB). Comme indiqué précédemment, le diamètre moyen des cristaux est généralement compris entre 100 nm et 1200 nm, de préférence compris entre 400 nm et 1200 nm, plus préférentiellement entre 500 nm et 1200 nm, de manière tout à fait préférée entre 550 nm et 1200 nm, très avantageusement entre 600 nm et 1200 nm, bornes incluses.

Cette observation MEB permet également de confirmer la présence de phase non zéolithique comprenant par exemple du liant résiduel (non converti lors de l'étape de zéolithisation) ou toute autre phase amorphe dans les agglomérés.

Dans le présent document, on emploie l'appellation « diamètre moyen en nombre » ou bien « taille », notamment pour les éléments cristallins de zéolithe et pour les adsorbants zéolithiques. La méthode de mesure de ces grandeurs est explicitée plus loin dans la description.

L'agglomération et la mise en forme (étape a) peuvent être réalisées selon toutes les techniques connues de l'homme de l'art, telles qu'extrusion, compactage, agglomération sur assiette granulatrice, tambour granulateur, atomisation et autres.

Les proportions de liant d'agglomération (voir définition plus loin) et de zéolithe mises en œuvre sont typiquement celles de l'art antérieur, c'est-à-dire de 5 parties à 20 parties en poids de liant pour 95 parties à 80 parties en poids de zéolithe.

Les agglomérés issus de l'étape a), qu'ils soient sous forme de billes, d'extrudés ou autres, ont en général un diamètre moyen en nombre, ou leur longueur (plus grande dimension lorsqu'ils ne sont pas sphériques), comprise entre 0,1 mm et 1 mm, et en particulier comprise entre 0,1 mm et 0,6 mm et de préférence entre 0,2 mm et 0,55 mm, bornes incluses.

À l'issue de l'étape a) les agglomérés les plus fins peuvent être éliminés par cyclonage et/ou tamisage et/ou les agglomérés trop gros par tamisage ou concassage, dans le cas d'extrudés, par exemple.

Le liant compris dans le matériau aggloméré zéolithique de la présente invention comprend, et de préférence consiste en, une argile ou un mélange d'argiles. Ces argiles sont de préférence choisies parmi les kaolins, kaolinites, nacrites, dickites, halloysites, attapulgites, sépiolites, montmorillonites, bentonites, illites et métakaolins, ainsi que les mélanges de deux ou plusieurs d'entre elles en toutes proportions.

Dans le cas de l'étape de zéolithisation le liant d'agglomération mis en œuvre à l'étape a) contient au moins 80% de préférence, au moins 90%, de préférence encore au moins 95%, plus particulièrement au moins 96%, en poids, d'au moins une argile zéolithisable et peut également contenir d'autres liants minéraux tels que bentonite, attapulgite, et autres. Par argile zéolithisable, on entend une argile ou un mélange d'argiles qui sont susceptibles d'être converties en matière zéolithique, le plus souvent par action d'une solution basique alcaline. L'argile zéolithisable appartient en général à la famille des kaolins (tels que par exemple kaolinites, nacrites, dickites, halloysites) et/ou des métakaolins.

Parmi les additifs éventuellement mis en œuvre à l'étape a), on peut trouver une source de silice de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Lors de l'étape a), outre les éléments cristallins de zéolithe FAU et le liant, d'autres additifs peuvent également être ajoutés, par exemple des additifs destinés à faciliter l'agglomération ou à améliorer le durcissement, ainsi que d'autres additifs connus de l'homme du métier.

En particulier si le liant d'agglomération contient une ou plusieurs argiles zéolithisables, la calcination permet de transformer l'argile zéolithisable, typiquement le kaolin, en méta-kaolin qui peut après être converti en zéolithe lors de l'étape de zéolithisation (étape b)). Le principe en est exposé dans « Zeolite Molecular Sieves » de D. W. Breck, John Wiley and Sons, New York, (1973), p. 314-315.

La zéolithisation éventuelle du liant d'agglomération est pratiquée selon toute méthode maintenant bien connue de l'homme du métier et peut par exemple être réalisée par immersion du produit issu de l'étape a) dans une solution basique alcaline, en général aqueuse, par exemple une solution aqueuse d'hydroxyde de sodium et/ou d'hydroxyde de potassium.

En règle générale, la concentration de la solution alcaline de zéolithisation est de préférence comprise entre 0,5 M et 5 M. La zéolithisation s'opère de préférence à chaud, à une température supérieure à la température ambiante, et typiquement à des températures de l'ordre de 80°C à 100°C. La durée du processus de zéolithisation est généralement comprise entre quelques dizaines de minutes et quelques heures, de préférence entre environ 1 heure et 8 heures.

De manière préférée et afin d'assurer une zéolithisation complète du liant sans altérer la cristallinité des cristaux de zéolithe présents on préférera mettre en contact les adsorbants avec une solution de soude froide puis effectuer une montée en température graduelle jusqu'à la température de 80°C-100°C.

De la même façon, la concentration en soude pourra être maintenue à la même concentration ou pourra être augmentée de façon graduelle afin de maintenir une cristallinité maximale des cristaux initiaux et assurer une conversion maximale du liant zéolithisable.

La ou les éventuelle(s) étape(s) d'échange(s) cationique(s) c) s'effectue(nt) selon les méthodes classiques connues de l'homme du métier, et le plus souvent par mise en contact des agglomérés issus de l'étape a) avec un sel du ou des cation(s) correspondants à l'échange cationique souhaité. L'opération d'échange cationique est généralement réalisée en solution aqueuse à une température comprise entre la température ambiante et 100°C, et de préférence comprise entre 80°C et 100°C.

On préfère opérer avec un large excès molaire de solution aqueuse du cation souhaité par rapport aux cations de la zéolithe que l'on souhaite échanger, avantageusement en procédant par échanges successifs, afin d'obtenir le taux d'échange désiré.

Après la ou les étape(s) d'échange(s) cationique(s), on procède ensuite à un lavage, généralement et de préférence à l'eau, puis d'un séchage de l'aggloméré ainsi obtenu.

L'activation qui suit le séchage, est conduite de manière classique, selon les méthodes connues de l'homme du métier, par exemple à une température en général comprise entre 100°C et 400°C, de préférence entre 200°C et 300°C pendant une durée déterminée en fonction de la teneur en eau et de la perte au feu souhaitées, typiquement de 1 heure à 6 heures.

## TECHNIQUES DE CARACTERISATION

Granulométrie des cristaux de zéolithe :

L'estimation du diamètre moyen en nombre des éléments (i.e. cristaux) de zéolithe de type FAU utilisées à l'étape a) et des éléments (i.e. cristaux) de zéolithe Y contenus dans les agglomérés est réalisée par observation au microscope électronique à balayage (MEB).

Afin d'estimer la taille des particules (i.e. cristaux) de zéolithe sur les échantillons, on effectue un ensemble de clichés à un grossissement d'au moins 5000. On mesure ensuite le diamètre d'au moins 200 particules à l'aide d'un logiciel dédié, par exemple le logiciel Smile View de l'éditeur LoGraMi. La précision est de l'ordre de 3%. La mesure de l'histogramme constitué à partir des dites mesures de diamètre permet en même temps la détermination de la déviation standard $\sigma$ de sa distribution.

Analyse chimique des agglomérés adsorbants zéolithiques - rapport Si/Al et taux d'échange :

Une analyse chimique élémentaire du produit final obtenu à l'issue des étapes a) à e) décrites précédemment, peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde une incertitude de mesure inférieure à 0,4% en poids.

Ces analyses chimiques élémentaires permettent à la fois de vérifier le rapport atomique Si/Al de la zéolithe utilisée au sein de l'aggloméré et le rapport atomique Si/Al du produit final obtenu à l'issue des étapes a) à e) décrites précédemment, et de vérifier la qualité de l'échange ionique décrit à l'étape c). Dans la description de la présente invention, l'incertitude de mesure du rapport atomique Si/Al est de $\pm$ 5%.

La qualité de l'échange ionique est liée au nombre de mole d'oxyde de sodium, $Na_2O$, restant dans l'aggloméré zéolithique après échange. Plus précisément, le taux d'échange par un cation est estimé en évaluant le rapport entre le nombre de moles d'oxyde dudit cation et le nombre de moles de l'ensemble des autres oxydes de cations présents.

Par exemple, dans le cas d'un aggloméré adsorbant zéolithique contenant des cations sodium et lithium, le taux d'échange du cation lithium est estimé en évaluant le rapport entre le nombre de moles d'oxyde de lithium, $Li_2O$, et le nombre de moles de l'ensemble ($Li_2O$ + $Na_2O$).

Il est à noter que les teneurs en différents oxydes sont donnés, en pourcentage en poids par rapport au poids total de l'aggloméré adsorbant zéolithique anhydre.

Granulométrie des adsorbants zéolithiques :

La détermination du diamètre moyen en nombre des adsorbants zéolithiques obtenus à l'issus de l'étape a) d'agglomération et de mise en forme est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra.

Le diamètre moyen en nombre est ensuite calculé à partir de la distribution granulométrique en appliquant la norme ISO 9276-2:2001. Dans le présent document, on emploie l'appellation « diamètre moyen en nombre » ou bien « taille » pour les agglomérés zéolithiques. La précision est de l'ordre de 0,01 mm pour la gamme de taille d'agglomérés de l'invention.

Résistance mécanique des adsorbants zéolithiques :

La résistance à l'écrasement d'un lit d'adsorbants zéolithiques tels que décrits dans la présente invention est caractérisée selon la méthode Shell série SMS1471-74 (Shell Method Series SMS1471-74 « Determination of Bulk Crushing Strength of Catalysts. Compression-Sieve Method »), associée à l'appareil « BCS Tester » commercialisé par la société Vinci Technologies. Cette méthode, initialement destinée à la caractérisation de catalyseurs de 3 mm à 6 mm est basée sur l'utilisation d'un tamis de 425 $\mu$m qui va permettre notamment de séparer les fines créées lors de l'écrasement. L'utilisation d'un tamis de 425 $\mu$m reste adaptée pour des particules de diamètre supérieur à 1,6 mm, mais doit être adaptée selon la granulométrie des agglomérés que l'on cherche à caractériser.

Les agglomérés de la présente invention, généralement sous forme de billes ou d'extrudés, ont en général un diamètre moyen en nombre ou une longueur, i.e. plus grande dimension dans le cas des agglomérés non sphériques, comprise entre 0,2 mm et 2 mm, et en particulier comprise entre 0,1 mm et 1,0 mm et de préférence entre 0,1 mm et 0,6 mm, bornes incluses. Par conséquent, un tamis de 100 $\mu$m est utilisé à la place du tamis de 425 $\mu$m mentionné dans la méthode Shell standard SMS1471-74.

Le protocole de mesure est le suivant : un échantillon de 20 cm$^3$ d'adsorbants agglomérés, préalablement tamisé avec le tamis adapté (100 $\mu$m) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm$^3$ de billes d'acier afin de mieux répartir la force exercée par le piston sur les agglomérés d'adsorbants (utilisation de billes de 2 mm de diamètre pour des particules de forme sphérique de diamètre strictement inférieur à 1,6 mm). Les fines obtenues aux différents paliers de pression sont séparées par tamisage (tamis adapté de 100 $\mu$m) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

Phase non zéolithique des adsorbants zéolithiques :

Le taux de phase non zéolithique PNZ, par exemple liant résiduel non zéolithisé ou toute autre phase amorphe, après zéolithisation est calculé selon l'équation suivante :

$$PNZ = 100 - \Sigma \ (PZ)$$

où PZ représente la somme des quantités des fractions zéolithiques Y au sens de l'invention.

La quantité des fractions zéolithiques Y (taux de cristallinité) est mesurée par analyse par diffraction de rayons X, connue de l'homme du métier sous l'acronyme DRX. Cette analyse est réalisée sur un appareil de la marque Bruker, puis la quantité des fractions zéolithiques Y est évaluée au moyen du logiciel TOPAS de la société Bruker.

Volume microporeux :

La cristallinité des agglomérés est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction.

Préalablement à l'adsorption, l'adsorbant zéolithique est dégazé entre 300°C et 450°C pendant une durée comprise entre 9 heures et 16 heures, sous vide (P < 6,7.10⁻⁴ Pa). La mesure de l'isotherme d'adsorption d'azote à 77 K est ensuite effectuée sur un appareil de type ASAP 2020 M de Micromeritics, en prenant au moins 35 points de mesure à des pressions relatives de rapport $P/P_0$ compris entre 0,002 et 1.

Volume total des macropores et des mésopores :

Les volumes macroporeux Vma et mésoporeux Vme, la densité de grain dg et la porosité $\varepsilon_p$, de type macro-porosité et mésoporosité, sont mesurés par porosimétrie par intrusion de mercure. Un porosimètre à mercure type Autopore® 9500 de Micromeritics est utilisé pour analyser la répartition du volume poreux contenu dans les macropores et dans les mésopores.

La méthode expérimentale, décrite dans le manuel opératoire de l'appareil faisant référence à la norme ASTM D4284-83, consiste à placer un échantillon d'adsorbant (adsorbant zéolithique sous forme d'agglomérés à mesurer) (de perte au feu connue) préalablement pesé, dans une cellule du porosimètre, puis, après un dégazage préalable (pression d'évacuation de 30 μm de mercure pendant au moins 10 min), à remplir la cellule avec du mercure à une pression donnée (0,0036 MPa), et ensuite à appliquer une pression croissante par palier jusqu'à 400 MPa afin de faire pénétrer progressivement le mercure dans le réseau poreux de l'échantillon, en prenant au moins 15 paliers de pression jusqu'à 0,2 MPa, et en appliquant ensuite des incréments de 0,1 MPa jusqu'à 1 MPa, puis 0,5 MPa jusqu'à 10 MPa, puis 2 MPa jusqu'à 30 MPa, puis 5 MPa jusqu'à 180 MPa, et enfin 10 MPa jusqu'à 400 MPa.

La relation entre la pression appliquée et la dimension caractéristique du seuil d'entrée de pore (correspondant à un diamètre apparent de pore) est établie en utilisant l'équation de Laplace-Young et en supposant une ouverture de pore cylindrique, un angle de contact entre le mercure et la paroi des pores de 140° et une tension de surface du mercure de 485 dynes cm⁻¹. Les incréments de volume ΔVi de mercure introduit à chaque palier de pression Pi sont enregistrés, ce qui permet ensuite de tracer le volume cumulé de mercure introduit en fonction de la pression appliquée V(Pi), ou en fonction du diamètre apparent des pores V(li). On fixe à 0,2 MPa la valeur à partir de laquelle le mercure remplit tous les vides inter-granulaires, et on considère qu'au-delà le mercure pénètre dans les pores de l'adsorbant. Le volume de grain Vg est alors calculé en soustrayant le volume cumulé de mercure à cette pression (0,2 MPa) au volume de la cellule du porosimètre, et en divisant cette différence par la masse de l'adsorbant équivalent anhydre, c'est-à-dire la masse dudit matériau corrigée de la perte au feu. La densité de grain dg est l'inverse du volume de grain Vg défini précédemment.

Le volume macroporeux Vma de l'adsorbant est défini comme étant le volume cumulé de mercure introduit à une pression comprise entre 0,2 MPa et 30 MPa, correspondant au volume contenu dans les pores de diamètre apparent supérieur à 50 nm. Le volume mésoporeux Vme de l'adsorbant est défini comme étant le volume cumulé de mercure introduit à une pression comprise entre 30 MPa et 400 MPa. La méthode de mesure du volume poreux par intrusion de mercure ne permettant pas d'accéder au volume microporeux, le volume poreux total Vtot tel que mesuré par intrusion de mercure, correspond à la somme des volumes macroporeux Vma et mésoporeux Vme.

Dans le présent document, les volumes macroporeux et mésoporeux Vma et Vme, ainsi que leur somme (volume poreux total Vtot), des adsorbants zéolithiques, exprimés en cm³ g⁻¹, sont ainsi mesurés par porosimétrie par intrusion de mercure et rapportés à la masse de l'échantillon en équivalent anhydre, c'est-à-dire la masse dudit adsorbant corrigée de la perte au feu. La densité de grain dg est exprimée en g cm⁻³ et se réfère à la masse de l'échantillon en équivalent anhydre.

La porosité $\varepsilon_p$ de type macroporosité et mésoporosité, est le produit de la densité de grain dg par la somme des volumes macroporeux et mésoporeux Vma et Vme :

$$\varepsilon p \ = \ dg \times (Vma \ + \ Vme)$$

Perte au feu des adsorbants zéolithiques :

La perte au feu est déterminée en atmosphère oxydante, par calcination de l'échantillon à l'air à une température de 900°C ± 25°C, en suivant le mode opératoire décrit dans la norme NF EN 196-2 (avril 2006). L'écart type de mesure est inférieur à 0,1%.

Exemples

Exemple 1 : Préparation des agglomérés

On prépare deux adsorbants (Agglomérés 1, comparatif et Aggloméré 2, selon l'invention) comme décrit ci-dessous, à partir d'une poudre de zéolithe faujasite de type Y, dont la taille moyenne des cristaux est de 0,5 µm. Les déviations standard respectives de ces cristaux sont de 0,23 µm, et 0,60 µm. Préparation de l'Aggloméré 1 (selon l'invention)

On prépare un mélange homogène et on agglomère 800 g de cristaux de zéolithe Y, de déviation standard de 0,23 µm, avec 160 g de kaolin (exprimés en équivalent calciné) et 195 g de silice colloïdale vendue sous la dénomination commerciale Klebosol™ 30N50 (contenant 30% en poids de $SiO_2$ et 0,5% en poids de $Na_2O$) avec la quantité d'eau qui permet l'extrusion du mélange. Les extrudés sont séchés, puis calcinés à 550°C (cuisson de l'argile) sous courant d'azote pendant 2 heures, et enfin concassés de manière à récupérer des agglomérés dont le diamètre moyen en nombre est égal à 0,5 mm.

Les agglomérés obtenus comme décrit ci-dessus (20 g) sont placés dans un réacteur en verre muni d'une double enveloppe régulée à une température de 90°C $\pm$ 1°C puis on ajoute 275 mL d'une solution aqueuse d'hydroxyde de sodium de concentration de 1,0 M, et on laisse le milieu réactionnel sous agitation pendant une durée de 3 heures 30 minutes.

On procède ensuite au lavage des agglomérés en 3 opérations successives de lavage à l'eau suivi de la vidange du réacteur. On s'assure de l'efficacité du lavage en mesurant le pH final des eaux de lavage qui est compris entre 10,0 et 10,5.

Les agglomérés sont ensuite séchés à 80°C pendant 2 heures et enfin activés à 400°C pendant 2 heures sous courant d'azote.

Préparation de l'Aggloméré 2 (comparatif)

On prépare un mélange homogène et on agglomère 800 g de cristaux de zéolithe de déviation standard de 0,60 µm, avec 145 g de kaolin (exprimés en équivalent calciné) et 180 g de silice colloïdale vendue sous la dénomination commerciale Klebosol™ 30N50 (contenant 30% en poids de $SiO_2$ et 0,5% en poids de $Na_2O$) avec la quantité d'eau qui permet l'extrusion du mélange. Les extrudés sont séchés, puis calcinés à 550°C (cuisson de l'argile) sous courant d'azote pendant 2 heures, et enfin concassés de manière à récupérer des agglomérés dont le diamètre moyen en nombre est égal à 0,5 mm.

Les agglomérés obtenus comme décrit ci-dessus (20 g) sont placés dans un réacteur en verre muni d'une double enveloppe régulée à une température de 95°C $\pm$ 1°C puis on ajoute 275 mL d'une solution aqueuse d'hydroxyde de sodium de concentration de 1,15 M, et on laisse le milieu réactionnel sous agitation pendant une durée de 3 heures.

On procède ensuite au lavage des agglomérés en 3 opérations successives de lavage à l'eau suivi de la vidange du réacteur. On s'assure de l'efficacité du lavage en mesurant le pH final des eaux de lavage qui est compris entre 10,0 et 10,5.

Les agglomérés sont ensuite séchés à 80°C pendant 2 heures et enfin activés à 400°C pendant 2 heures sous courant d'azote.

Caractéristiques des Agglomérés 1 à 2

Les valeurs de porosité de grain $\varepsilon_p$ et les déviations standard $\sigma$ des cristaux dans les agglomérés finaux, et telles que mesurées pour les Agglomérés 1 à 2 sont données dans le Tableau 1 ci-dessous.

L'Aggloméré 1, à base de cristaux de NaY, présente une taille moyenne de cristaux mesurée sur l'aggloméré final de 0,71 µm, l'Aggloméré 2, à base de cristaux de NaY, présente une taille moyenne de cristaux mesurée sur l'aggloméré final de 0,65 µm

La perte au feu mesurée comme décrit précédemment est de 2,5% $\pm$ 0,1%.

Exemple 2 : Test de perçage sur adsorbants des exemples 1 et 2

Un test de perçage (chromatographie frontale) est ensuite réalisé sur ces adsorbants pour évaluer leur efficacité. La quantité d'adsorbant utilisée pour ce test est d'environ 26 g.

Le mode opératoire pour obtenir les courbes de perçage est le suivant :

- Remplissage de la colonne par le tamis et mise en place dans le banc de test.

- Remplissage par le solvant à température ambiante.

- Montée progressive à la température d'adsorption sous flux de solvant (5 cm3.min-1).

- Injection de solvant à 30 $cm^3.min^{-1}$ lorsque la température d'adsorption est atteinte.

- Permutation solvant/charge pour injecter la charge (30 cm$^3$.min$^{-1}$).

- L'injection de la charge est ensuite maintenue un temps suffisant pour atteindre l'équilibre thermodynamique (c'est-à-dire jusqu'à ce que la concentration en solvant dans l'effluent soit nulle).

- Collecte et analyse de l'effluent du perçage.

Le solvant utilisé est le para-diéthylbenzène. La composition de la charge est la suivante :

Métaxylène : 45% poids

Orthoxylène : 45% poids

Iso-octane : 10% poids (celui-ci est utilisé comme traceur pour l'estimation des volumes non-sélectifs et n'intervient pas dans la séparation)

Le test est réalisé avec une température d'adsorption de 140°C. La pression est suffisante pour que la charge reste en phase liquide, soit 1 MPa. La vitesse superficielle (débit/section de la colonne) de circulation du liquide à la température du test est d'environ 1,2 cm.s$^{-1}$ pour l'ensemble des tests.

La sélectivité du méta-xylène (MX) par rapport à l'ortho-xylène (OX) ($\alpha_{MX/OX}$) est calculée à partir des volumes adsorbés qMX et qOX des composés MX et OX (ces derniers étant déterminés par bilan matière à partir de l'analyse de l'effluent du perçage) et de la composition de la charge (charge dans laquelle la fraction volumique des composés est yMX et yOX) :

$$\alpha_{MX/OX} = \frac{q_{MX}}{q_{OX}} \frac{y_{OX}}{y_{MX}}$$

Les résultats du perçage sont consignés dans le Tableau 1 ci-dessous :

-- Tableau 1 --

| Adsorbant | Porosité de grain $\varepsilon_p$ (%) | Déviation standard $\sigma$ ($\mu m$) | sélectivité MX/OX | Capacité d'adsorption (%) | HEPT MX (%) |
|---|---|---|---|---|---|
| exemple 1 (invention) | 36 | 0,19 | 1,83 | 13.3 | 8.63 |
| exemple 2 (comparatif) | 27 | 0,35 | 1,84 | 13,2 | 10,2 |

Légende

Capacité d'adsorption exprimée en % (cm$^3$ de C8-aromatiques adsorbés par cm$^3$ de colonne)

HEPT = Hauteur Équivalente à un Plateau Théorique mesurée sur le méta-xylène exprimée en % de longueur de colonne

MX = Méta-Xylène ; OX = Ortho-Xylène

On observe que l'utilisation de l'adsorbant selon l'invention permet de réduire considérablement la Hauteur Équivalente à un Plateau Théorique mesurée pour le méta-xylène, indiquant une amélioration du transfert de matière. Cette amélioration sur le transfert de matière se traduit de manière proportionnelle sur la productivité du procédé.

**Revendications**

1. Adsorbant zéolithique aggloméré, comprenant au moins une zéolithe faujasite de rapport atomique Si/Al strictement supérieur à 1,50, avantageusement compris entre 1,50 et 6,50 bornes exclues, de préférence entre 2,00 et 6,00 bornes incluses, de préférence encore entre 2,00 et 4,00, bornes incluses et de manière encore plus préférée entre 2,20 et 2,80 bornes incluses, caractérisé d'une part en ce que la porosité de grain dudit adsorbant est comprise entre 25% et 45 %, de préférence entre 30% et 45 %, de préférence encore entre 32% et 45% et de manière très préférée entre 35% et 45%, de manière particulièrement avantageuse entre 36% et 45%, bornes incluses, et d'autre part en ce que la déviation standard $\sigma$ de la distribution de taille de cristaux dans ledit aggloméré est inférieure à 0,30 $\mu m$, de préférence comprise entre 0,05 $\mu m$ et 0,30 $\mu m$, de préférence encore entre 0,05 $\mu m$ et 0,28 $\mu m$, et de manière tout à fait préférée entre 0,1 μm et 0,28 μm, de préférence entre toutes entre 0,1 μm et 0,25 μm, bornes incluses,

ledit adsorbant comprenant des cristaux de zéolithe de diamètre moyen en nombre compris entre 100 nm et 1200 nm, bornes incluses, et

la fraction massique de zéolithe FAU dans ledit adsorbant est supérieure ou égale à 80% par rapport au poids total d'adsorbant.

2. Adsorbant zéolithique aggloméré selon la revendication 1 dans lequel ledit adsorbant comprend des cristaux de zéolithe de diamètre moyen en nombre compris entre 400 nm et 1200 nm, plus préférentiellement entre 500 nm et 1200 nm, de manière tout à fait préférée entre 550 nm et 1200 nm, très avantageusement entre 600 nm et 1200 nm, bornes incluses.

3. Adsorbant zéolithique aggloméré selon l'une des revendications 1 ou 2, **caractérisé en ce qu'**il se présente sous la forme de billes de diamètre moyen compris entre 100 μm et 1000 μm, de préférence entre 100 μm et 600 μm, de préférence encore 200 μm et 550 μm, bornes incluses.

4. Adsorbant zéolithique aggloméré selon l'une des revendications 1 à 3 dans lequel ladite au moins une zéolithe FAU-Y présente un rapport atomique Si/Al compris entre 1,50 et 6,50 bornes exclues, de préférence entre 2,00 et 6,00 bornes incluses, de préférence encore entre 2,00 et 4,00, bornes incluses et de manière encore plus préférée entre 2,20 et 2,80 bornes incluses.

5. Adsorbant zéolithique aggloméré selon l'une des revendications 1 à 4, comprenant un ou plusieurs cation(s), de préférence alcalin(s) ou alcalino-terreux, de préférence encore choisi(s) parmi lithium, sodium, baryum, calcium et potassium, seuls ou en mélanges de deux ou plusieurs d'entre eux, et de manière tout à fait préférée choisi(s) parmi lithium, sodium et calcium, seuls ou en mélanges de deux ou plusieurs d'entre eux.

6. Adsorbant zéolithique aggloméré selon l'une des revendications 1 à 5, **caractérisé en ce qu'**il contient du sodium en une teneur, exprimée en poids d' oxyde de sodium ($Na_2O$) dans l'adsorbant zéolithique aggloméré supérieure à 5%, de préférence encore supérieure à 8%, de manière très préférée supérieure à 10%, en poids par rapport à la masse totale de l'adsorbant, et avantageusement, ladite teneur en oxyde de sodium est comprise entre 10% et 17%, et typiquement entre 11% et 13%, bornes incluses, en poids par rapport au poids total de l'adsorbant.

7. Adsorbant zéolithique aggloméré selon l'une des revendications 1 à 6, dans lequel la teneur totale en cations autres que le lithium, le sodium, le potassium, le calcium et le baryum est inférieure à 2%, de préférence comprise entre 0 et 1%, bornes incluses, cette teneur totale étant exprimée en masse d'oxydes desdits cations, par rapport à la masse totale de l'adsorbant zéolithique.

8. Adsorbant zéolithique aggloméré selon l'une quelconque des revendications précédentes, dans lequel aucune structure zéolithique autre que la structure faujasite, de préférence aucune structure zéolithique autre que la structure faujasite Y, n'est détectée par diffraction des rayons X

9. Adsorbant selon l'une quelconque des revendications précédentes, dans lequel la zéolithe FAU est une zéolithe Y.

10. Utilisation d'un adsorbant selon l'une quelconque des revendications 1 à 9, dans les procédés de :

- séparation de coupes d'isomères aromatiques en C8 et notamment des xylènes,
- séparation d'isomères de toluène substitué tels que nitrotoluène, diéthyltoluène, toluènediamine, et autres,
- séparation des crésols,
- séparation des alcools polyhydriques.

11. Utilisation selon la revendication 10, pour la séparation de méta-xylène à partir de coupes d'isomères aromatiques à 8 atomes de carbone.

12. Procédé de séparation de *méta*-xylène à partir de coupes d'isomères aromatiques à 8 atomes de carbone, utilisant comme agent d'adsorption du méta-xylène, un adsorbant zéolithique aggloméré selon l'une des revendications 1 à 9, en phase liquide ou en phase gazeuse.

13. Procédé de séparation de méta-xylène à partir de coupes d'isomères d'hydrocarbures aromatiques contenant 8 atomes de carbone, selon la revendication 12, en phase liquide, par adsorption du méta-xylène en présence d'un désorbant.

14. Procédé selon l'une quelconque des revendications 12 ou 13 de type lit mobile simulé, de préférence à contre-courant simulé.


**Patentansprüche**

1. Agglomeriertes zeolithisches Adsorptionsmittel, umfassend mindestens einen Faujasit-Zeolith mit einem Si/Al-Atomverhältnis streng größer als 1,50, vorteilhafterweise zwischen 1,50 und 6,50, Grenzwerte ausgeschlossen, vorzugsweise zwischen 2,00 und 6,00, Grenzwerte eingeschlossen, noch weiter bevorzugt zwischen 2,00 und 4,00, Grenzwerte eingeschlossen, und noch weiter bevorzugt zwischen 2,20 und 2,80, Grenzwerte eingeschlossen, **dadurch gekennzeichnet, dass** einerseits die Kornporosität des Adsorptionsmittels zwischen 25 % und 45 %, vorzugsweise zwischen 30 % und 45 %, beträgt, noch weiter bevorzugt zwischen 32 % und 45 % und sehr bevorzugt zwischen 35 % und 45 %, besonders vorteilhaft zwischen 36 % und 45 %, Grenzwerte eingeschlossen, liegt, und andererseits dadurch, dass die Standardabweichung $\sigma$ der Kristallgrößenverteilung in dem Agglomerat kleiner als 0,30 $\mu$m ist, vorzugsweise zwischen 0,05 $\mu$m und 0,30 $\mu$m, noch weiter bevorzugt zwischen 0,05 $\mu$m und 0,28 $\mu$m, und ganz besonders bevorzugt zwischen 0,1 $\mu$m und 0,28 $\mu$m, ganz besonders bevorzugt zwischen 0,1 $\mu$m und 0,25 $\mu$m, Grenzwerte eingeschlossen, liegt,

   wobei das Adsorptionsmittel Zeolithkristalle mit einem zahlenmittleren Durchmesser zwischen 100 nm und 1200 nm, Grenzwerte eingeschlossen, umfasst und
   der Massenanteil an FAU-Zeolith in dem Adsorptionsmittel größer oder gleich 80 %, bezogen auf das Gesamtgewicht des Adsorptionsmittels, ist.

2. Agglomeriertes zeolithisches Adsorptionsmittel nach Anspruch 1, wobei das Adsorptionsmittel Zeolithkristalle mit einem zahlenmittleren Durchmesser zwischen 400 nm und 1200 nm, weiter bevorzugt zwischen 500 nm und 1200 nm, ganz besonders bevorzugt zwischen 550 nm und 1200 nm, sehr vorteilhaft zwischen 600 nm und 1200 nm, Grenzwerte eingeschlossen, umfasst.

3. Agglomeriertes zeolithisches Adsorptionsmittel nach einem der Ansprüche 1 oder 2, **dadurch gekennzeichnet, dass** es in Form von Kugeln mit einem mittleren Durchmesser zwischen 100 $\mu$m und 1000 $\mu$m, vorzugsweise zwischen 100 $\mu$m und 600 $\mu$m, weiter bevorzugt 200 $\mu$m und 550 $\mu$m, Grenzwerte eingeschlossen, vorliegt.

4. Agglomeriertes zeolithisches Adsorptionsmittel nach einem der Ansprüche 1 bis 3, wobei der mindestens eine FAU-Y-Zeolith ein Si/Al-Atomverhältnis zwischen 1,50 und 6,50, Grenzwerte ausgeschlossen, vorzugsweise zwischen 2,00 und 6,00, Grenzwerte eingeschlossen, noch weiter bevorzugt zwischen 2,00 und 4,00, Grenzwerte eingeschlossen, und noch weiter bevorzugt zwischen 2,20 und 2,80, Grenzwerte eingeschlossen, aufweist.

5. Agglomeriertes zeolithisches Adsorptionsmittel nach einem der Ansprüche 1 bis 4, umfassend ein oder mehrere Kationen, vorzugsweise Alkali- oder Erdalkali-Kationen, weiter bevorzugt ausgewählt aus Lithium, Natrium, Barium, Calcium und Kalium, allein oder in Mischungen von zwei oder mehr davon, und ganz besonders bevorzugt ausgewählt aus Lithium, Natrium und Calcium, allein oder in Mischungen von zwei oder mehr davon.

6. Agglomeriertes zeolithisches Adsorptionsmittel nach einem der Ansprüche 1 bis 5, **dadurch gekennzeichnet, dass** es Natrium in einem Gehalt, ausgedrückt als Gewicht an Natriumoxid (Na$_2$O), in dem agglomerierten zeolithischen Adsorptionsmittel von mehr als 5 Gew.-%, weiter bevorzugt mehr als 8 Gew.-%, sehr bevorzugt mehr als 10 Gew.-% bezogen auf die Gesamtmasse des Adsorptionsmittels, enthält und der Natriumoxidgehalt vorteilhafterweise zwischen 10 Gew.-% und 17 Gew.-% und typischerweise zwischen 11 Gew.-% und 13 Gew.-%, Grenzwerte eingeschlossen, bezogen auf das Gesamtgewicht des Adsorptionsmittels, liegt.

7. Agglomeriertes zeolithisches Adsorptionsmittel nach einem der Ansprüche 1 bis 6, wobei der Gesamtgehalt an Kationen, die von Lithium, Natrium, Kalium, Calcium und Barium verschieden sind, weniger als 2 % beträgt und vorzugsweise zwischen 0 und 1 %, Grenzwerte eingeschlossen, liegt, wobei dieser Gesamtgehalt als Masse der Oxide dieser Kationen, bezogen auf die Gesamtmasse des zeolithischen Adsorptionsmittels, ausgedrückt wird.

8. Agglomeriertes zeolithisches Adsorptionsmittel nach einem der vorhergehenden Ansprüche, wobei durch Röntgenbeugung keine andere zeolithische Struktur als die Faujasitstruktur, vorzugsweise keine andere zeolithische Struktur als die Faujasit-Y-Struktur, detektiert wird.

9. Adsorptionsmittel nach einem der vorhergehenden Ansprüche, wobei es sich bei dem FAU-Zeolith um einen Y-Zeolith handelt.

10. Verwendung eines Adsorptionsmittels nach einem der Ansprüche 1 bis 9 bei Verfahren zur:

  - Trennung von Schnitten von aromatischen C8-Isomeren und insbesondere Xylolen,
  - Trennung von Isomeren von substituiertem Toluol wie Nitrotoluol, Diethyltoluol, Toluoldiamin und anderen,
  - Trennung von Kresolen,
  - Trennung von mehrwertigen Alkoholen.

11. Verwendung nach Anspruch 10 zur Abtrennung von *meta*-Xylol aus Schnitten von aromatischen Isomeren mit 8 Kohlenstoffatomen.

12. Verfahren zur Abtrennung von *meta*-Xylol aus Schnitten von aromatischen Isomeren mit 8 Kohlenstoffatomen unter Verwendung eines agglomerierten zeolithischen Adsorptionsmittels nach einem der Ansprüche 1 bis 9 als Adsorptionsmittel für *meta*-Xylol in der Flüssigphase oder in der Gasphase.

13. Verfahren zur Abtrennung von *meta*-Xylol aus Schnitten von aromatischen Kohlenwasserstoffisomeren mit 8 Kohlenstoffatomen nach Anspruch 12 in der Flüssigphase durch Adsorption des *meta*-Xylols in Gegenwart eines Desorptionsmittels.

14. Verfahren nach einem der Ansprüche 12 oder 13 vom Typ simuliertes Fließbett, vorzugsweise mit simuliertem Gegenstrom.

## Claims

1. Agglomerated zeolitic adsorbent, comprising at least one faujasite zeolite with an Si/Al atomic ratio strictly of greater than 1.50, advantageously of between 1.50 and 6.50, limits excluded, preferably between 2.00 and 6.00, limits included, more preferably between 2.00 and 4.00, limits included, and more preferably still between 2.20 and 2.80, limits included, characterized, on the one hand, in that the grain porosity of said adsorbent is of between 25% and 45%, preferably between 30% and 45%, more preferably between 32% and 45% and very preferably between 35% and 45%, particularly advantageously between 36% and 45%, limits included, and, on the other hand, in that the standard deviation $\sigma$ of the crystal size distribution in said agglomerate is less than 0.30 $\mu$m, preferably of between 0.05 $\mu$m and 0.30 $\mu$m, more preferably between 0.05 $\mu$m and 0.28 $\mu$m, and entirely preferably between 0.1 $\mu$m and 0.28 $\mu$m, particularly preferably between 0.1 $\mu$m and 0.25 $\mu$m, limits included, said adsorbent comprising zeolite crystals with a number-average diameter of between 100 nm and 1200 nm, limits included, and
the fraction by weight of FAU zeolite in said adsorbent is greater than or equal to 80%, with respect to the total weight of adsorbent.

2. Agglomerated zeolitic adsorbent according to Claim 1, in which said adsorbent comprises zeolite crystals with a number-average diameter of between 400 nm and 1200 nm, more preferentially between 500 nm and 1200 nm, entirely preferably between 550 nm and 1200 nm, very advantageously between 600 nm and 1200 nm, limits included.

3. Agglomerated zeolitic adsorbent according to either of Claims 1 and 2, **characterized in that** it is provided in the form of beads with a mean diameter of between 100 $\mu$m and 1000 $\mu$m, preferably between 100 $\mu$m and 600 $\mu$m, more preferably between 200 $\mu$m and 550 $\mu$m, limits included.

4. Agglomerated zeolitic adsorbent according to one of Claims 1 to 3, in which said at least one FAU-Y zeolite exhibits an Si/Al atomic ratio of between 1.50 and 6.50, limits excluded, preferably between 2.00 and 6.00, limits included, more preferably between 2.00 and 4.00, limits included, and more preferably still between 2.20 and 2.80, limits included.

5. Agglomerated zeolitic adsorbent according to one of Claims 1 to 4, comprising one or more cations, preferably alkali metal or alkaline earth metal cations, more preferably chosen from lithium, sodium, barium, calcium and potassium, alone or as mixtures of two or more of them, and entirely preferably chosen from lithium, sodium and calcium, alone or as mixtures of two or more of them.

6. Agglomerated zeolitic adsorbent according to one of Claims 1 to 5, **characterized in that** it contains sodium in a content, expressed by weight of sodium oxide ($Na_2O$) in the agglomerated zeolitic adsorbent, of greater than 5%, more preferably of greater than 8%, very preferably of greater than 10%, by weight, with respect to the total weight of the adsorbent, and advantageously said sodium oxide content is of between 10% and 17% and typically between 11% and 13%, limits included, by weight, with respect to the total weight of the adsorbent.

7. Agglomerated zeolitic adsorbent according to one of Claims 1 to 6, in which the total content of cations other than lithium, sodium, potassium, calcium and barium is less than 2%, preferably between 0% and 1%, limits included, this total content being expressed by weight of oxides of said cations, with respect to the total weight of the zeolitic adsorbent.

8. Agglomerated zeolitic adsorbent according to any one of the preceding claims, in which no zeolitic structure other than the faujasite structure, preferably no zeolitic structure other than the faujasite Y structure, is detected by X-ray diffraction.

9. Adsorbent according to any one of the preceding claims, in which the FAU zeolite is a Y zeolite.

10. Use of an adsorbent according to any one of Claims 1 to 9, in processes for:

- separation of cuts of aromatic C8 isomers and in particular of xylenes,
- separation of isomers of substituted toluene, such as nitrotoluene, diethyltoluene, toluenediamine and others,
- separation of cresols,
- separation of polyhydric alcohols.

11. Use according to Claim 10, for the separation of meta-xylene from cuts of aromatic isomers having 8 carbon atoms.

12. Process for the separation of meta-xylene from cuts of aromatic isomers having 8 carbon atoms, using, as agent for adsorption of meta-xylene, an agglomerated zeolitic adsorbent according to one of Claims 1 to 9, in the liquid phase or in the gas phase.

13. Process for the separation of meta-xylene from cuts of aromatic hydrocarbon isomers containing 8 carbon atoms according to Claim 12, in the liquid phase, by adsorption of the meta-xylene in the presence of a desorbent.

14. Process according to either one of Claims 12 and 13 of simulated moving bed type, preferably simulated counter-current moving bed type.

**EP 4 076 732 B1**

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4306107 A
- US 4326092 A
- US 5382747 A
- US 5900523 A
- US 7728187 B
- FR 2998808
- FR 2889698
- FR 2889699
- TW 201107032
- US 2985589 A
- WO 2008009845 A

- WO 2014090771 A
- WO 2018002174 A
- US 20090326308 A
- US 7208651 B
- US 6136198 A
- US 2009326308 A
- US 10125064 B
- US 2007224113 A
- US 10071914 B
- WO 2009081022 A

**Littérature non-brevet citée dans la description**

- **GOMES et al.** *Adsorption*, 2006, vol. 12, 375
- **D. W. BRECK**. Zeolite Molecular Sieves. John Wiley and Sons, 1973, 314-315

- Determination of Bulk Crushing Strength of Catalysts. Compression-Sieve Method. *Shell Method Series SMS1471-74*